# EUROPÄISCHE PATENTSCHRIFT

(11) **EP 1 520 562 B1**
(45) Veröffentlichungstag und Bekanntmachung des Hinweises auf die Patenterteilung: **27.06.2007**
(21) Anmeldenummer: 04090365.0
(22) Anmeldetag: 20.09.2004
(51) Int. Cl.: A61F 2/46, B01F 11/00

(54) **Vorrichtung zum Mischen und Austragen von flüssigen und pulverförmigen Materialien für die medizinische Verwendung**
Device for mixing and delivering of liquid and powdery substances for medical use.
Dispositif pour mélanger et distribuer des substances liquides et pulvérulentes pour l'utilisation médicale.

(30) Priorität: 01.10.2003 DE 10345646
(43) Veröffentlichungstag der Anmeldung: 06.04.2005
(73) Patentinhaber: Biomet Deutschland GmbH, 14167 Berlin (DE)
(72) Erfinder: Nagaraj, Hutchappa Nandeesh, Jayanagar, Bangalore-560011 (IN)
(74) Vertreter: Maikowski, Michael

(56) Entgegenhaltungen:
- EP-A- 0 692 229
- WO-A-97/18031
- WO-A-03/008080
- US-A- 6 017 349

## Beschreibung

Die Erfindung betrifft eine Vorrichtung zum Mischen und Austragen von flüssigen und pulverförmigen Materialien für die medizinische Verwendung, welche die Merkmale des Oberbegriffs des Anspruchs 1 aufweist.

Bei verschiedenen medizinischen Behandlungsverfahren wird ein künstlich hergestelltes Material in den Körper des Patienten eingebracht. So wird beispielsweise bei der Vertebroplastie künstlich hergestellter, zunächst flüssiger Knochenzement direkt in eine geschwächte Knochenmatrix injiziert, um diese zu stützen und dadurch Schmerzen zu vermindern, die durch deformierte Knochen oder den Verlust an Knochenmasse verursacht wurden. Da das künstlich hergestellte Material in den menschlichen oder tierischen Körper eingebracht wird und dort über einen langen Zeitraum verbleibt, sind die Anforderungen hinsichtlich einer guten Verträglichkeit sowie einer sterilen Zusammensetzung und Handhabung sehr hoch.

Typische Knochenzemente setzen sich aus einer flüssigen Komponente, oftmals Monomerlösungen von Methylmethacrylat (MMA) mit kleineren Mengen anderer Monomere und Zusatzstoffe sowie einer festen, pulverförmigen Komponente zusammen, die oftmals im Wesentlichen aus einem Polymer wie beispielsweise Polymethylmethacrylat (PMMA) besteht. Andere Mischprodukte setzen sich typischerweise aus einem mineralischen Pulver wie beispielsweise Kalziumphosphat oder Kalziumsulfat mit gegebenenfalls weiteren Zusatzstoffen oder Wirkstoffen wie Antibiotika und einer Flüssigkeit in Form einer wässrigen Lösung von Salzen bzw. einer anderen Pufferlösung, insbesondere einer Natriumphosphatlösung zusammen. Es sind auch Mischungen aus zwei pastösen Komponenten denkbar, die für eine Anwendung im menschlichen Körper geeignete Eigenschaften aufweisen können.

Allen diesen künstlich hergestellten Materialien ist gemeinsam, dass zur Herstellung eine flüssige Ausgangskomponente mit einem weiteren oftmals festen, gegebenenfalls auch flüssigen Material intensiv durchmischt werden muss. Nach der Durchmischung ist dann innerhalb einer kurzen Zeitspanne eine Verwendung und Verarbeitung des Mischprodukts möglich, bevor die gewünschte Verfestigung und Aushärtung des Mischprodukts einsetzt.

Es sind zahlreiche Vorrichtungen bekannt, die für das Vermischen der Materialien und für deren Einbringung in den menschlichen Körper geeignet sind. Zumindest der letzte Schritt, nämlich die Austragung der miteinander vermischten Materialien, muss natürlich unter sterilen Bedingungen direkt im Operationssaal erfolgen. Da nach dem erstmaligen Durchmischen der beiden Ausgangsmaterialien oftmals nur sehr kurze Zeit bis zur bestimmungsgemäß Verwendung des fertig gemischten Produkts zur Verfügung steht, ist es wünschenswert, dass auch der Mischvorgang unter sterilen Bedingungen durchgeführt werden kann.

Das durch Mischung erzeugte Endprodukt kann - auch in Abhängigkeit der jeweils verwendeten Ausgangsmaterialien - während der medizinischen Anwendung eine über einen weiten Bereich unterschiedliche Viskosität aufweisen. Eine gezielte und kontrollierte Austragung des Endprodukts während einer Operation kann insbesondere bei hochviskosen Endprodukten, die gegebenenfalls noch feinkörnige Zusatzstoffe enthalten können, mit Schwierigkeiten verbunden sein.

Es ist aus der Praxis ein Mischbehälter bekannt, bei dem die in einem Mischzylinder zusammengeführten Ausgangsmaterialien mittels eines handbetätigbaren Mischkolbens intensiv durchmischt werden können. Zum Austragen der vermischten Materialien wird der Mischzylinder in ein Handhabungsgerät eingesetzt, das einen kontrollierten Austrag des vermischten Endprodukts ermöglicht. Die zunächst getrennt gelagerten Ausgangsstoffe sind während des Mischvorgangs in dem Mischzylinder dicht verschlossen, so dass die handbetätigte Durchmischung und das anschließende Austragen unter sterilen Bedingungen, beispielsweise im Operationssaal erfolgen können.

Die zu mischenden Ausgangskomponenten müssen unmittelbar vor dem Durchmischen in den Mischzylinder eingefüllt werden, was üblicherweise nicht unter sterilen Bedingungen und außerhalb des Operationssaales erfolgt. Der befüllte Mischzylinder muss dann sterilisiert werden, um das Durchmischen und die Verwendung des Mischprodukts im Operationssaal zu ermöglichen.

Durch die Verwendung des zusätzlichen Handhabungsgerätes wird zwar das Austragen auch hochviskoser Endprodukte erleichtert, jedoch ist das Einsetzen des Mischzylinders in das Handhabungsgerät nach der erfolgten Durchmischung zeitaufwendig und umständlich und die Herstellung und vor jeder Verwendung erforderliche Sterilisation des Handhabungsgeräts vergleichsweise aufwendig. Auf Grund der geringen Herstellungskosten und des unverhältnismäßig hohen Reinigungsaufwands werden Mischzylinder in Verbindung mit Misch- und Austragskolben üblicherweise als Einwegprodukte konzipiert und hergestellt. Die vergleichsweise hohen Herstellungkosten von den voranstehend beschriebenen Handhabungsgeräten, die mit den verwendeten Materialien nicht unmittelbar in Kontakt kommen, führen jedoch dazu, dass das Handhabungsgerät wiederverwendbar ausgeführt ist und nach jeder Anwendung gereinigt und sterilisiert werden muss.

Es ist auch eine Vorrichtung zum Mischen und Austragen von Knochenzementen bekannt (EP 0 692 229 B1), die eine getrennte Lagerung der Ausgangskomponenten unter sterilen Bedingungen ermöglicht. Bei dieser Vorrichtung müssen jedoch nach Beendigung des Mischvorgangs und vor dem Austragen des durch Mischung erhaltenen Knochenzements Betätigungsvorrichtungen für den Mischkolben entfernt und Mittel zur Betätigung des Austragskolbens angebracht werden. Dabei wird der Austragskolben üblicherweise als axial verschiebbarer Presskolben mittels einer an dem Austragskolben zu befestigenden Druckstange in den Mischzylinder eingedrückt und verdrängt dadurch den dort befindlichen Knochenzement, der durch einen ebenfalls nachträglich an dem Mischzylinder anzubringenden Austragsstutzen austritt.

Die Vorrichtung ist kostengünstig herstellbar und eignet sich zur Verwendung als Einwegprodukt. Die Handhabung einer solchen Vorrichtung ist jedoch wegen der erforderlichen Umrüstung nach dem Mischvorgang und vor der Austragung zeitaufwendig und umständlich und erlaubt insbesondere bei hochviskosen Mischprodukten keine dosierbare, bzw. kontrollierte Austragung des Knochenzements

Ferner ist aus der WO 97/18031 eine gattungsgemäße Vorrichtung bekannt.

Aufgabe der vorliegenden Erfindung ist es eine möglichst einfache, kostengünstig herstellbare Vorrichtung so auszugestalten, dass eine intensive Durchmischung der Ausgangsmaterialien und deren kontrollierter Austrag bei gleichzeitig möglichst einfacher Handhabung ermöglicht werden. Die gesamte Vorrichtung sollte wirtschaftlich sinnvoll als Einwegprodukt herstellbar sein und verwendet werden können. Zumindest während des Mischvorgangs und der Austragung der gemischten Materialien sollte eine unerwünschte Kontamination der sowohl Umgebung als auch der verwendeten Materialien ausgeschlossen sein.

Diese Aufgabe wird erfindungsgemäß mit den kennzeichnen den Merkmalen des Anspruchs 1 gelöst.

Dadurch wird erreicht, dass die Handhabung der Vorrichtung während des Mischvorgangs und des Auftragens wesentlich vereinfacht wird. Es sind keine zusätzlichen Maßnahmen notwendig, um beispielsweise die Vorrichtung nach dem Mischvorgang so umzurüsten, dass ein einfaches Austragen des Mischprodukts ermöglicht wird. Wegen der vereinfachten Handhabung reduziert sich die während der Vorbereitung und Verwendung des Mischprodukts notwendige Zeit merklich, was dazu führt, dass auch die Dauer des operativen Eingriffs verkürzt und die Belastungen für den Patienten wie für den ausführenden Arzt vermindert werden können.

Da die Vorrichtung Mittel zur getrennten Aufnahme und Lagerung der zu mischenden Komponenten aufweist, können die Komponenten bereits lange vor der vorgesehenen Verwendung in die Vorrichtung eingebracht werden. Die Befüllung der Vorrichtung könnte beispielsweise direkt nach deren Herstellung unter sterilen Bedingungen erfolgen und Vorrichtungen, die je nach Anwendung mit verschiedenen Mischungskomponenten befüllt wurden, in dieser Form angeboten und vertrieben werden. Auch wenn die Befüllung getrennt von der Herstellung und dem Vertrieb der Vorrichtung erfolgt, können die getrennt aufgenommenen Materialien in der Vorrichtung oftmals über einen ausreichend langen Zeitraum bis zur Verwendung gelagert werden, so dass die Notwendigkeit einer Befüllung unmittelbar vor oder während Verwendung bei einer Operation im Operationssaal entfällt.

Es ist vorgesehen, dass die Vorrichtung zwei voneinander dicht trennbare Kammern aufweist, in welchen zwei zu mischende Materialien getrennt aufnehmbar und lagerbar sind. In den getrennten Kammern können flüssige oder pulverförmige Materialien ohne zusätzliche Verpackungsmittel, bzw. Schutzhüllen wie beispielsweise Folienbeutel oder Kartonagen auch über einen längeren Zeitraum gelagert werden. Vor dem Zusammenfügen und Mischen der Materialien ist es nicht erforderlich, zuerst eine Verpackung zu öffnen, bevor eine Durchmischung vorgenommen werden kann. Dadurch wird die Handhabung insbesondere unter sterilen Bedingungen wesentlich vereinfacht.

Es ist vorgesehen, dass der Austragskolben im Inneren eine Lagerkammer zur Aufnahme einer der zu mischenden Materialien aufweist. Die Lagerkammer im Inneren des Austragskolbens ist ein von dem Innenraum des Mischzylinders getrennter Hohlraum. Der Innenraum des Mischzylinders bildet dann ohne zusätzliche Maßnahmen eine zweite, von der Lagerkammer im Austragskolben getrennte Kammer.

Die Lagerkammer im Innenraum des Austragskolbens kann dabei im Wesentlichen hohlzylindrisch sein, wobei die Lagerkammer an der Außenseite durch die zylindrische Außenwand des Austragskolbens und an der Innenseite durch die innenliegende, axiale Lagerung der Betätigungsstange des Mischkolbens begrenzt ist. Das damit zur Verfügung stehende Volumen reicht für die Verwendung der Vorrichtung mit den gängigen Ausgangsmaterialien aus. Zweckmäßigerweise wird dabei ein flüssiges Ausgangsmaterial in die Lagerkammer im Innenraum des Austragskolbens eingefüllt und dort gelagert, da eine weitgehend restfreie Entleerung der Lagerkammer des Austragskolbens sich bei flüssigen Materialen einfacher bewerkstelligen lässt.

Vorteilhafterweise ist vorgesehen, dass der Austragskolben eine verschließbare Öffnung aufweist, über welche im geöffneten Zustand das in der Lagerkammer des Austragskolbens befindliche Material in den Mischzylinder gelangen kann. Durch Öffnen dieser verschließbaren Öffnung wird eine Verbindung zwischen der Lagerkammer in dem Austragskolben und dem Mischzylinder hergestellt, über den das bislang im Austragskolben gelagerte Ausgangsmaterial in den Mischzylinder gelangt. In den meisten Fällen wird es dabei ausreichen, die Vorrichtung für kurze Zeit mit dem Mischzylinder nach unten so zu halten, dass die Flüssigkeit aus dem höher liegenden Austragskolben infolge der Schwerkraft nach unten in den Mischzylinder eintritt. Bei Verwendung eines pastösen Ausgangsmaterials kann es aber auch zweckmäßig sein, die Entleerung der Lagerkammer des Austragskolbens beispielsweise mechanisch oder durch einen zusätzlich erzeugten Überdruck zu unterstützen.

Vorzugsweise ist die verschließbare Öffnung des Austragskolbens über einen an dem dem Mischzylinder abgewandten Ende des Austragskolbens angeordneten Verschlussmechanismus verschließbar. Das Öffnen und Verschließen des Austragskolbens zu Beginn des Mischvorgangs, um die bis dahin gelagerten Ausgangsmaterialien zusammenzuführen, lässt sich dann in einfacher Weise von der frei zugänglichen Seite der Vorrichtung aus vornehmen und kontrollieren.

Der Verschlussmechanismus weist zweckmäßigerweise Handhabungsmittel zur einfachen Betätigung des Verschlussmechanismus auf. So könnte beispielsweise das Öffnen oder Schließen der verschließbaren Öffnung durch Umlegen eines kleinen Hebels oder Betätigen eines Bajonettverschlusses vorgenommen werden.

Einer vorteilhaften Ausführung des Erfindungsgedankens zufolge sind Belüftungsöffnungen der Lagerkammer des Austragskolbens vorgesehen, die eine kontrollierte Befüllung und Entleerung der Lagerkammer ermöglichen. Beim Befüllen der Lagerkammer mit einem flüssigen Ausgangsmaterial muss die dabei verdrängte Luft aus dem Innenraum entweichen können. In gleicher Weise muss bei dem Entleeren der Lagerkammer zu Beginn des Mischvorgangs Luft in das frei werdende Volumen des Innenraums nachströmen können, um einen anderenfalls entstehenden Unterdruck in der Lagerkammer zu vermeiden, der eine weitere Entleerung des Innenraums erschwert oder ganz verhindert.

Die zum Befüllen bzw. Entleeren der Lagerkammer verwendeten Öffnungen können ausreichend groß dimensioniert werden, um gleichzeitig als Belüftungsöffnungen wirken zu können. Sollte die Größe der zum Befüllen und Entleeren verwendeten Öffnungen nicht ausreichen, so können zusätzliche verschließbare Belüftungsöffnungen beispielsweise an dem Mischzylinder abgewandten Ende des Austragungskolbens vorgesehen sein.

Vorzugsweise ist vorgesehen, dass die Austragsöffnung an einer Endfläche des Mischzylinders angeordnet ist und die Betätigungsstange des Mischkolbens und die Mittel zur Betätigung des Austragskolbens an dem entgegengesetzten Ende des Mischzylinders angeordnet sind. Die ganze Vorrichtung kann demnach ähnlich wie eine Spritze konstruiert sein und verwendet werden. Für die meisten chirurgischen Eingriffe ist es von Vorteil, wenn die Austragsöffnung, aus welcher das in den Körper des Patienten einzubringende Mischprodukt austritt, möglichst weit entfernt von Handhabungs- und Betätigungselementen der verwendeten Vorrichtung angeordnet ist.

Gemäß einer bevorzugten Ausführung des Erfindungsgedankens ist vorgesehen, dass der Austragskolben mit dem Mischzylinder über ein Schraubgewinde in Eingriff steht und durch ein Verdrehen des Auftragskolbens relativ zum Mischzylinder der Austragskolben in dem Mischzylinder verschiebbar und die im Mischzylinder befindlichen durchmischten Materialien austragbar sind. Es hat sich gezeigt, dass in vielen Fällen das manuelle Eindrücken bzw. Verschieben des Austragskolbens in dem Mischzylinder nicht die gewünschte Präzision bei der Dosierung während des Austragens ermöglicht. So kann insbesondere bei einem hochviskosen Mischprodukt der erforderliche Druck während des Austragens des Materials derart groß sein, dass ein gleichmäßiger Austrag und eine an dem Ort des operativen Eingriffs möglichst bewegungsfreie Positionierung der Vorrichtung nicht in jedem Fall gewährleistet werden können.

Durch die Verwendung eines Schraubgewindes, welches die manuelle Drehbewegung in eine axiale Verschiebung des Austragskolbens umsetzt, wird der zum Austragen erforderliche Kraftaufwand erheblich reduziert. Über die Steigung des Gewindes kann die für ein vollständiges Austragen des Mischprodukts notwendige Anzahl der Umdrehungen vorgegeben werden, wobei sich der erforderliche Kraftaufwand mit zunehmender Anzahl von Umdrehungen reduziert. Nur in den seltensten Fällen ist ein rascher Austrag nahezu der gesamten Mischproduktmenge notwendig und deshalb nur eine geringe Anzahl von Umdrehungen zum vollständigen Austrag des Mischprodukts wählbar.

Da während des Austrags des Mischprodukts aus der Vorrichtung keine manuellen Kräfte in axialer Richtung ausgeübt werden müssen, lässt sich die Vorrichtung und insbesondere deren Austragsöffnung wesentlich leichter und gleichmäßiger dort positionieren, wo das austretende Mischprodukt in den Körper des Patienten eingebracht werden soll.

Vorteilhafterweise ist vorgesehen, dass der Mischzylinder ein Außengewinde aufweist, welches mit einem Innengewinde eines den Mischzylinder teilweise umgebenden Hohlzylinders in Eingriff steht, wobei der Hohlzylinder mit dem Austragskolben starr verbunden ist. Auf diese Weise wird vermieden, dass die Innenseite des Mischzylinders ein Gewinde aufweist. Die demzufolge glatte, zylinderförmige Innenfläche des Mischzylinders ohne Vorsprünge aus Ausnehmungen gewährleistet eine einfache Abdichtung des Innenraums des Mischzylinders und verhindert dadurch eine unerwünschte Kontamination der in dem Mischzylinder befindlichen Materialien.

An der Außenseite des Mischzylinders kann sich das Außengewinde dagegen ohne weiteres bis in einen Bereich des Mischzylinders erstrecken, der für eine intensive Durchmischung der Ausgangskomponenten mittels des im Inneren des Mischzylinders hin und her bewegten Mischkolbens genutzt wird. Die Länge des Bewegungsgewindes kann damit unabhängig von dem im Mischzylinder vorgesehenen Mischvolumen so vorgegeben sein, dass ein Verkanten der Gewindeflächen des Mischzylinders und des diesen umgebenden Hohlzylinders weitestgehend ausgeschlossen ist.

Gemäß einer Ausgestaltung des Erfindungsgedankens ist vorgesehen, dass der Hohlzylinder an seiner Außenfläche im Bereich der starren Verbindung mit dem Austragskolben Handhabungselemente aufweist. Mehrere Erhebungen und/oder Vertiefungen, welche beispielsweise wie eine Flügelmutter gestaltet sein können, gewährleisten eine sichere Handhabung und Verdrehung des Hohlzylinders auch unter ungünstigen Bedingungen. Der Hohlzylinder kann auch an seiner gesamten Außenfläche Handhabungselemente aufweisen, beispielsweise in Längsrichtung verlaufende, entlang des Umfangs beabstandete, abgerundete Vertiefungen und/oder Ausformungen. Auch ist es denkbar, die Außenseite des Hohlzylinders der besseren Handhabung wegen mit einem beispielsweise elastischen oder mit einer rauen Oberfläche versehenen Material zu überziehen.

Gemäß einer Ausgestaltung des Erfindungsgedankens ist vorgesehen, dass die Vorrichtung im Bereich der Austragungsöffnung so mit einem Standfuß verbindbar ist, dass die Austragsöffnung dicht verschlossen ist. Dadurch wird sichergestellt, dass die Austragsöffnung während des Mischvorgangs dicht verschlossen ist und kein Material austreten kann, während gleichzeitig die Vorrichtung auf einer festen Unterlage aufrecht stehend positioniert werden kann, um die Betätigung der dann nach oben herausragenden Betätigungsstange des Mischkolbens in einfacher und kräfteschonender Weise zu erlauben. Der Standfuß ermöglicht weiterhin eine sichere Aufbewahrung der bereits befüllten Vorrichtung bis zu deren Verwendung.

Vorteilhaferweise ist vorgesehen, dass eine Kanüle mit der Austragsöffnung verbindbar ist. Beispielsweise bei der Vertebroplastie muss der zunächst flüssige Knochenzement in das Innere einer degenerierten, bzw. geschwächten Knochenstruktur eingebracht werden, um diese zu stützen und zu entlasten. Durch eine Kanüle hindurch kann der in der Vorrichtung vorbereitete Knochenzement nahezu punktgenau an dem gewünschten Ort in den Knochen eingebracht werden, ohne dass umfangreiche vorbereitende operative Maßnahmen notwendig sind.

Vorteilhafterweise ist vorgesehen, dass während des Mischvorgangs oder unmittelbar danach ein Unterdruck in dem Mischzylinder erzeugbar ist. Es hat sich gezeigt, dass eine unerwünschte Porenbildung in dem Mischprodukt zumindest wesentlich herabgesetzt werden kann, wenn während des Durchmischens der Materialien ein Unterdruck herrscht. Dazu kann vorgesehen sein, dass der Mischzylinder entweder direkt oder über den mit dem Mischzylinder in Verbindung stehenden Innenraum des Austragskolbens mit einer Vakuumleitung verbunden wird. Durch geeignete Vorgabe des in der Vakuumleitung herrschenden Unterdrucks bzw. durch zusätzliche Ventile muss dabei sichergestellt werden, dass die zu mischenden Ausgangsmaterialien nicht ungewollt aus dem Mischzylinder abgesaugt werden.

Einer Ausgestaltung des Erfindungsgedankens zufolge ist vorgesehen, dass die Innenflächen der Lagerkammer des Austragskolbens und/oder des Mischzylinders mit einer Schutzschicht überzogen sind. Unabhängig von dem jeweiligen Material, aus welchem der Austragskolben bzw. die gesamte Vorrichtung hergestellt ist, verhindert die Schutzschicht auch bei längerer Lagerung der bereits eingefüllten Ausgangsmaterialien eine Wechselwirkung und damit eine Kontamination der Ausgangsmaterialien mit dem Material des Austragskolbens, bzw. des Mischzylinders. Auch wenn das eingefüllte Ausgangsmaterial keine besonders chemisch aggressive Zusammensetzung aufweist, so wird mit einer Schutzschicht auch die unvermeidbare Diffusion an den Kontaktflächen selbst bei längerer Lagerung der befüllten Vorrichtung weitgehend verhindert.

Weitere Ausgestaltungen des Erfindungsgedankens sind Gegenstand weiterer Unteransprüche. Im Folgenden wird ein Ausführungsbeispiel der Erfindung näher erläutert, welches in der Zeichnung dargestellt ist. Es zeigt:
Fig. 1 eine Seitenansicht eine Vorrichtung zum Mischen und Austragen von flüssigen und pulverförmigen Materialien für die medizinische Verwendung mit einer daran befestigten Kanüle,
Fig. 2 einen Schnitt längs der Linie II-II in Fig. 1,
Fig. 3 eine Seitenansicht der in Fig. 1 dargestellten Vorrichtung, wobei die Kanüle von einer Schutzkappe bedeckt ist,
Fig. 4 einen Schnitt längs der Linie IV-IV in Fig. 3,
Fig. 5 eine Seitenansicht der in Fig. 1 dargestellten Vorrichtung, wobei anstelle der Kanüle ein Standfuß mit der Vorrichtung verbunden ist,
Fig. 6 einen Schnitt der in der Fig. 5 dargestellten Vorrichtung entlang der Linie VI-VI,
Fig. 7 eine Seitenansicht eines Austragskolbens mit einem damit verbundenen Hohlzylinder,
Fig. 8 einen Schnitt durch den in Fig. 7 dargestellten Austragskolben entlang der Linie VIII-VIII,
Fig. 9 eine Seitenansicht eines Mischzylinders mit einem Außengewinde,
Fig. 10 einen Schnitte entlang der Linie X-X des in Fig. 9 dargestellten Mischzylinders,
Fig. 11 eine Ansicht der Stirnfläche eines durchbrochenen Mischkolbens,
Fig. 12 eine Seitenansicht des in den Fig. 5 und 6 in Verbindung mit der Vorrichtung dargestellten Standfußes und
Fig. 13 einen Schnitt des in Fig. 12 gezeigten Standfußes entlang der Linie XIII-XIII.

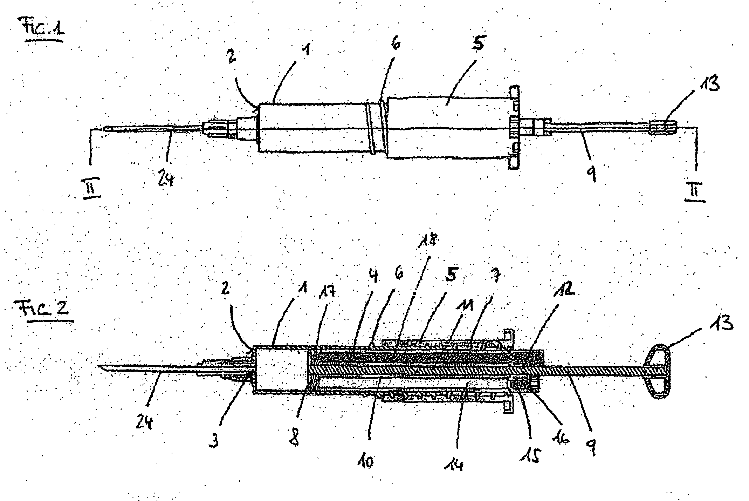

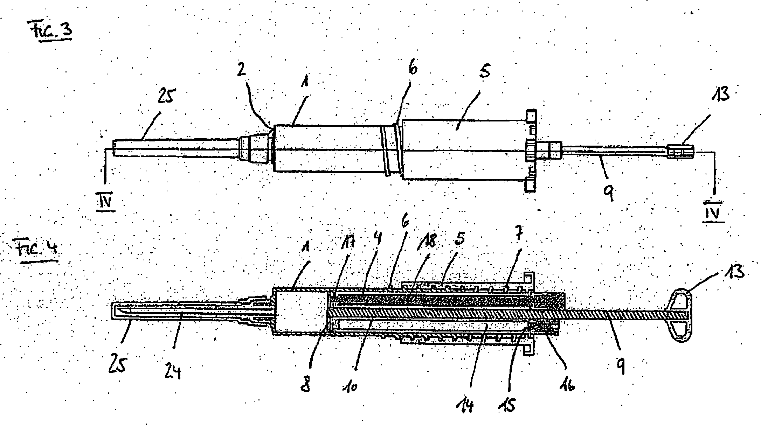

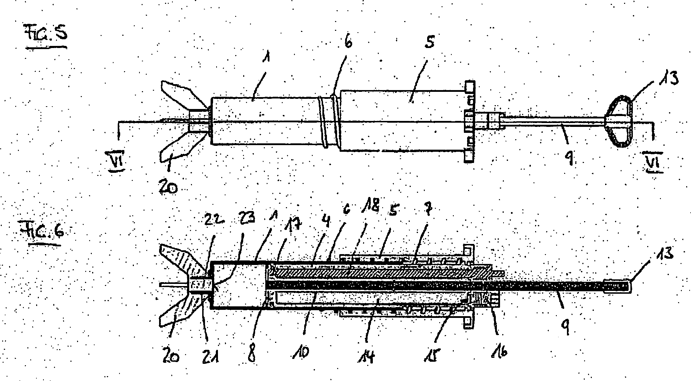

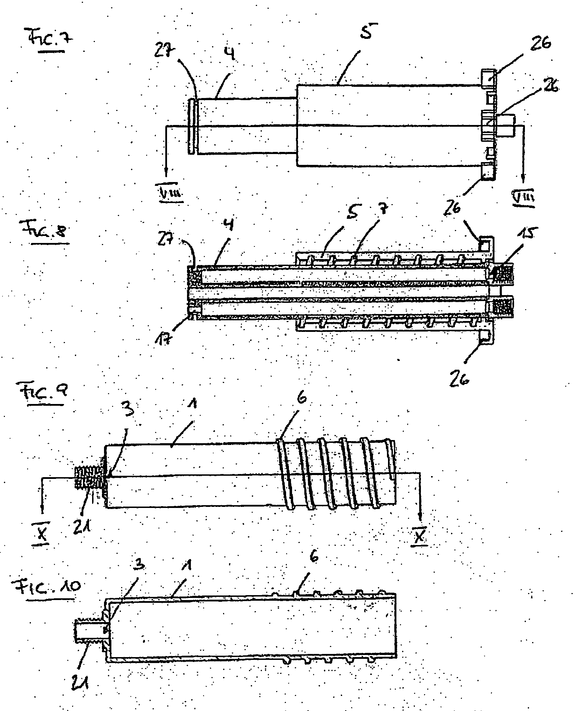

Die in den Fig. 1 bis 6 dargestellte Vorrichtung zum Mischen und Austragen von flüssigen und pulverförmigen Materialien für die medizinische Verwendung weist einen Mischzylinder 1 auf, an dessen einer Endfläche 2 eine Austragsöffnung 3 angeordnet ist. Das der Austragsöffnung 3 gegenüberliegende Ende des Mischzylinders 1 ist im Wesentlichen offen. Ein Austragskolben 4 ist axial verschiebbar in dem Mischzylinder 1 angeordnet, wobei der aus dem offenen Ende des Mischzylinders 1 herausragende Austragskolben 4 jenseits des Mischzylinders 1 mit einem den Mischzylinder 1 teilweise umgebenden Hohlzylinder 5 starr verbunden ist.

Der Mischzylinder 1 weist an seiner Außenseite ein Außengewinde 6 auf, welches mit einem daran angepassten Innengewinde 7 an der Innenseite des den Mischzylinder 1 teilweise umgebenden Hohlzylinders 5 in Eingriff steht. Durch ein Verdrehen des Hohlzylinders 5 relativ zu dem Mischzylinder 1 bewirkt das von dem Außengewinde 6 und dem Innengewinde 7 gebildete Bewegungsgewinde eine Umsetzung der Drehbewegung in eine entsprechende axiale Verschiebung des Hohlzylinders 5 und des damit starr verbundenen Austragskolbens 4. Durch Festhalten des Mischzylinders 1 und gleichzeitiges Drehen des Hohlzylinders 5 wird demzufolge eine axiale Verschiebung des Austragskolbens 4 im Inneren des Mischzylinders 1 bewirkt, so dass ein gleichmäßiger Austrag der sich im Inneren des Mischzylinders 1 befindenden, nicht dargestellten Materialien entsprechend der Verdrängung durch den vorrückenden Austragskolben 4 erfolgt.

Ein durchbrochener Mischkolben 8 ist zwischen der Austragsöffnung 3 und dem Austragskolben 4 axial und drehbar beweglich in dem Mischzylinder 1 angeordnet und eine Betätigungsstange 9 ist durch den Austragskolben 4 hindurch abgedichtet aus dem Mischzylinder 1 herausgeführt. Die Befestigungsstange 9 des Mischkolbens 8 ist in einer im Inneren des Austragskolbens 4 ausgebildeten Lagerhülse 10 axial und drehbar beweglich gelagert. Eine mit etwas Abstand zur Stirnfläche des Mischkolbens 8 angeordnete ringförmige Dichtung 11 entlang des Umfangs der Betätigungsstange bildet eine luftdichte Abdichtung des Innenraums des Mischzylinders 1 von der Umgebung und verhindert zuverlässig den Austritt von den in dem Mischzylinder 1 gelagerten oder vermischten Materialien. Eine ringförmige Ausnehmung 12 entlang des Umfangs der Betätigungsstange 9 bildet eine Sollbruchstelle, so dass nach erfolgtem Mischvorgang das herausragende Ende der Betätigungsstange 9 abgebrochen und entfernt werden kann, um die weitere Handhabung der Vorrichtung zu erleichtern. An dem herausragenden Ende der Betätigungsstange 9 befindet sich ein flügelmutterartig ausgebildeter Betätigungsgriff 13, der eine sichere und kraftvolle Hin- und Herbewegung des Mischkolbens 8 in dem Inneren des Mischzylinders 1 erleichtert, der über die Betätigungsstange 9 mit dem Betätigungsgriff 13 verbundenen ist.

Der Austragskolben 4 weist eine eine Lagerkammer 14 in Form einer im Wesentlichen hohlzylinderförmige Aussparung in seinem Innenraum auf, die zur Aufnahme der zu mischenden Materialien vor dem eigentlichen Mischvorgang dient. Der Austragskolben 4 weist weiterhin an dem dem Mischzylinder 1 abgewandten Ende eine verschließbare Befüllungsöffnung 15 zur Befüllung der Lagerkammer 14 des Austragskolbens 4 auf. Bei dem dargestellten Ausführungsbeispiel ist die Befüllungsöffnung 15 mittels eines eingeschraubten Stifts 16 dicht verschlossen.

Der Austragskolben 4 weist an seinem dem Innenraum des Mischzylinders 1 zugewandten Ende eine verschließbare Öffnung 17 auf, über welche im geöffneten Zustand die in der Lagerkammer 14 des Austragskolbens 4 befindliche Ausgangskomponente in den Mischzylinder 1 gelangen kann. Die verschließbare Öffnung 17 ist durch eine sich durch die Lagerkammer 14 des Austragskolbens 4 erstreckende Verschlussstange 18 verschlossen, deren eines Ende in die verschließbare Öffnung 17 ragt und diese dicht verschließt. Die Verschlussstange 18 ist an dem dem Mischzylinder 1 abgewandten Ende des Austragskolbens 4 axial verschiebbar gelagert. Anstelle des bei der dargestellten Ausführungsform gezeigten Gewindes 19, welches ein sicheres Öffnen und Verschließen der verschließbaren Öffnung 17 durch entsprechendes Ausdrehen bzw. Eindrehen der Verschlussstange 18 ermöglicht, könnte die Verschlussstange 18 auch mit einem Bajonettverschluss versehen sein, der sich über einen kleinen Hebel oder dergleichen betätigen lässt.

Die in den Fig. 1 bis 6 dargestellte Vorrichtung ermöglicht eine einfache Handhabung während des Befüllens der Vorrichtung mit den Ausgangsmaterialien, während des Mischvorgangs und des anschließenden Austrags der durchmischten Materialien.

Zum Befüllen der Vorrichtung wird zunächst der Austragskolben 4 samt dem damit verbundenen Mischkolben 8 vollständig aus dem Mischzylinder 1 herausgenommen. In den Innenraum des Mischzylinders 1 wird eine Komponente der zu mischenden Ausgangsmaterialien, üblicherweise ein pulverförmiges Material, eingefüllt. Der Innenraum des Mischzylinders 1 wird anschließend durch den Austragskolben 4 wieder dicht verschlossen. Der Austragskolben 4 wird dabei so weit in den Mischzylinder 1 eingeführt, dass das verbleibende Innenraumvolumen des Mischzylinders 1 ausreicht, um später auch die zweite Komponente der zu mischenden Materialien vollständig aufzunehmen und eine intensive Durchmischung der Materialien zu ermöglichen. Danach wird die Befüllungsöffnung 15 der Lagerkammer 14 im Austragskolben 4 geöffnet und die zweite, üblicherweise flüssige Komponente der zu mischenden Materialien in die Lagerkammer 14 des Austragskolbens 4 eingefüllt. Die Befüllungsöffnung 15 wird anschließend durch Eindrehen des Stifts 16 in die Befüllungsöffnung 15 wieder dicht verschlossen. Die befüllte und dicht verschlossene Vorrichtung kann nunmehr bis zur Verwendung unter sterilen Bedingungen außerhalb oder innerhalb des Operationssaales gelagert werden.

Vor Beginn des Mischvorgangs muss zunächst die Verschlussstange 18 gelöst werden und verschließbare Öffnung 17 freigegeben werden. In den meisten Fällen genügt bereits eine aufrechte Positionierung der Vorrichtung für kurze Zeit, um ein vollständiges Austreten der in der Lagerkammer 14 des Austragskolbens 4 befindlichen Flüssigkeit in den dann darunter befindlichen Mischzylinder 1 zu gewährleisten. Werden hochviskose Ausgangsmaterialien in den hohlzylindrischen Innenraum der Lagerkammer 14 des Austragskolbens 4 eingefüllt, so dass eine nicht restlose Entleerung vor Beginn des Mischvorgangs zu befürchten ist, so können zusätzliche, nicht dargestellte Mittel verwendet werden, um eine weitgehend restfreie Entleerung des Austragskolbens 4 sicherzustellen.

Nachdem die verschließbare Öffnung 17 des Austragskolbens 4 nach der Entleerung wieder verschlossen wurde, kann durch wiederholtes Verschieben des durchbrochenen Mischkolbens 8 im Innenraum 14 des Mischzylinders 1 eine intensive Durchmischung der nunmehr im Mischzylinder 1 zusammengeführten Komponenten vorgenommen werden.

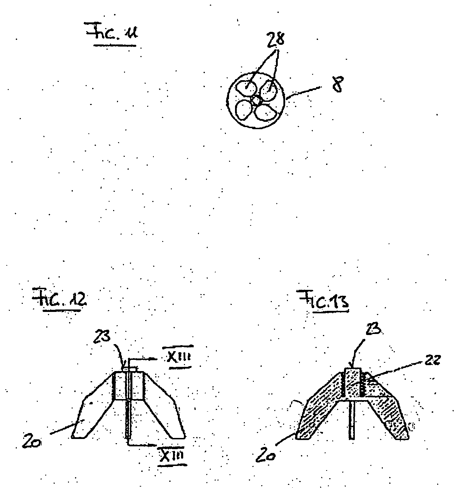

Dabei ist die Austragsöffnung 3 zweckmäßigerweise durch einen auch in den Fig. 5 und 6 dargestellten Standfuß 20 verschlossen. Der Standfuß 20 ist über ein Gewinde mit einem daran angepassten Anschlussstutzen 21 der Austragsöffnung 3 verbunden. Eine konzentrische Ausformung 22 ragt bei einem vollständig mit der Vorrichtung verbundenem Standfuß 20 so weit in den Anschlussstutzen 21 der Austragsöffnung 3 hinein, dass die Stirnseite 23 der konzentrischen Ausformung 22 bündig den Innenraum des Mischzylinders 1 abschließt. Auf diese Weise wird während des Mischvorgangs ein Totvolumen im Innenraum des Mischzylinders 1 vermieden, in welchem sich nur unvollständig durchmischte Mengen der Ausgangskomponenten sammeln könnten.

Die Vorrichtung kann mit dem Standfuß 20 auf eine ebene Unterlage aufrecht gestellt werden, so dass die Betätigungsstange 9 nach oben herausragt und der Betätigungsgriff 13 leicht zugänglich ist. Eine einfache Auf- und Abbewegung der Betätigungsstange 9 bewirkt dann ein entsprechende Verschiebung des durchbrochenen Mischkolbens 8 und damit die gewünschte Vermischung der Ausgangsmaterialien.

Nach ausreichender Durchmischung der Materialien kann der Standfuß 20 abgeschraubt und durch eine Kanüle 24 ersetzt werden. Durch ein Verdrehen des Hohlzylinders 5 relativ zu dem festgehaltenen Mischzylinder 1 wird der Austragskolben 4 zunehmend in den Mischzylinder 1 eingedrückt und verdrängt das darin befindliche, in den Fig. nicht dargestellte Mischprodukt, das durch die Kanüle 24 hindurch austritt. Der Austragskolben 4 schiebt während seines Vorrückens im Innenraum des Mischzylinders 1 den Mischkolben 8 vor sich her, ohne dass dies zu einer spürbaren Beeinträchtigung des Austragsvorgangs führt. Gegebenenfalls kann für eine einfachere Handhabung der Vorrichtung der nicht mehr benötigte Teil der Betätigungsstange 9 zusammen mit dem Betätigungsgriff 13 an der Sollbruchstelle 12 der Betätigungsstange 9 abgebrochen und entfernt werden.

Aus Sicherheitsgründen kann vor und während einer Operation bzw. im Anschluss daran bei der nicht benutzten Vorrichtung die Kanüle 24 mit einer Schutzkappe 25 bedeckt werden.

In den Fig. 7 bis 10 sind der Austragskolben 4 zusammen mit dem Hohlzylinder 5 sowie der Mischzylinder 1 zur Veranschaulichung noch einmal gesondert dargestellt. Der Hohlzylinder 5 weist an seiner Außenfläche im Bereich der starren Verbindung mit dem Austragskolben 4 Handhabungselemente in Form von mehreren, im Abstand zueinander angeordneten abgerundeten Ausformungen 26 auf. An seinem dem Mischzylinder 1 zugewandten Ende weist der Austragskolben 4 eine entlang des Umfangs umlaufende ringförmige Ausnehmung 27 auf, die zur Aufnahme eines Dichtrings oder ähnlicher Dichtmittel geeignet ist und den Innenraum des Mischzylinders 1 zuverlässig von der Umgebung abdichtet.

In Fig. 11 ist die Stirnseite des durchbrochenen Mischkolbens 8 dargestellt, so dass die Gestaltung und Anordnung der einzelnen Durchbrechungen 28 ersichtlich wird.

Zur anschaulichen Verdeutlichung ist in den Fig. 12 und 13 der in den Fig. 5 und 6 bereits gezeigte und in diesem Zusammenhang beschriebene Standfuß 20 in Alleinstellung dargestellt.

Die einzelnen Elemente der in den Fig. dargestellten Vorrichtung können überwiegend aus Polycarbonaten bzw. Polypropylen und im Spritzgussverfahren hergestellt werden. Sofern erforderlich, können beispielsweise die Betätigungsstange 9 oder der Stift 16 bzw. die Verschlussstange 18 aus Aluminium oder einem anderen Material ausreichender Festigkeit hergestellt sein. Für bestimmte Anwendungen kann es vorteilhaft oder erforderlich sein, dass die Innenflächen der Aussparung 14 des Austragskolbens 4 und des Mischzylinders 1 mit einer chemisch beständigen Schutzschicht, beispielsweise einer dünnen Teflon- oder Glasbeschichtung überzogen sind.

## Patentansprüche

1. Vorrichtung zum Mischen und Austragen von flüssigen und pulverförmigen Materialien für die medizinische Verwendung mit einem Mischzylinder (1), mit einem durchbrochenen Mischkolben (8), der in dem Mischzylinder (1) mittels einer Betätigungsstange (9) axial und drehbar beweglich ist, mit einer verschließbaren Austragsöffnung (3) am Mischzylinder (1),mit einem axial verschiebbaren Austragskolben (4), mit einem Mittel zur Betätigung des Austragskolbens (4), um durch ein Verschieben des Austragskolbens (4) im Mischzylinder (1) die durchmischten Materialien durch die Austragsöffnung (3) auszutragen, mit zwei voneinander dicht trennbaren Kammern zur getrennten Aufnahme und Lagerung von zwei zu mischenden Materialien,
**dadurch gekennzeichnet,**
**dass** der Austragskolben (4) im Inneren eine Lagerkammer (14) zur Aufnahme eines zu mischenden Materials aufweist.

2. Vorrichtung nach Anspruch 1, **dadurch gekennzeichnet, dass** in dem Mischzylinder (1) und in der Lagerkammer (14) im Inneren des Austragskolbens (4) zwei zu mischende Materialien getrennt aufnehmbar sind.

3. Vorrichtung nach Anspruch 2, **dadurch gekennzeichnet, dass** der Austragskolben (4) eine verschließbare Öffnung (17) aufweist, über welche im geöffneten Zustand das in der Lagerkammer (14) des Auftragskolbens (4) befindliche Material in den Mischzylinder (1) gelangen kann.

4. Vorrichtung nach Anspruch 3, **dadurch gekennzeichnet, dass** die verschließbare Öffnung (17) des Austragskolbens (4) über einen an dem dem Mischzylinder (1) abgewandten Ende des Austragskolbens (4) angeordneten Verschlussmechanismus betätigbar ist.

5. Vorrichtung nach Anspruch 4, **dadurch gekennzeichnet, dass** der Verschlussmechanismus eine an dem dem Mischzylinder (1) abgewandten Ende des Austragskolbens (4) axial verschiebbar gelagerte Verschlussstange (18) aufweist und durch Betätigung der Verschlussstange (18) die verschließbare Öffnung (17) freigegeben oder verschlossen werden kann.

6. Vorrichtung nach Anspruch 4 oder 5, **dadurch gekennzeichnet, dass** der Verschlussmechanismus Handhabungsmittel zur einfachen Betätigung aufweist.

7. Vorrichtung nach mindestens einem der vorhergehenden Ansprüche, **dadurch gekennzeichnet, dass** der Austragskolben (4) an dem dem Mischzylinder (1) abgewandten Ende eine verschließbare Befüllungsöffnung (15) zur Befüllung der Lagerkammer (14) des Austragskolbens (4) mit einem der zu mischenden Materialien aufweist.

8. Vorrichtung nach mindestens einem der vorhergehenden Ansprüche, **dadurch gekennzeichnet, dass** Belüftungsöffnungen der Lagerkammer (14) des Austragskolbens (4) vorgesehen sind, die eine kontrollierte Befüllung und Entleerung der Lagerkammer (14) ermöglichen.

9. Vorrichtung nach mindestens einem der vorhergehenden Ansprüche, **dadurch gekennzeichnet, dass** die Austragsöffnung (3) an einer Endfläche (2) des Mischzylinders (1) angeordnet ist und die Betätigungsstange (9) des Mischkolbens (8) und die Mittel zur Betätigung des Austragskolbens (4) an dem entgegengesetzten Ende des Mischzylinders (1) angeordnet sind.

10. Vorrichtung nach mindestens einem der vorhergehenden Ansprüche, **dadurch gekennzeichnet, dass** der Mischkolben (8) zwischen der Austragsöffnung (3) und dem Austragskolben (4) im Mischzylinder (1) angeordnet ist und die Betätigungsstange (9) des Mischkolbens (8) durch den Austragskolben (4) hindurch abgedichtet herausgeführt ist.

11. Vorrichtung nach mindestens einem der vorhergehenden Ansprüche, **dadurch gekennzeichnet, dass** der Austragskolben (4) mit dem Mischzylinder (1) über ein Gewinde in Eingriff steht und durch ein Verdrehen des Austragskolbens (4) relativ zum Mischzylinder (1) der Austragskolben (4) in dem Mischzylinder verschiebbar und die im Mischzylinder befindlichen durchmischten Materialien austragbar sind.

12. Vorrichtung nach mindestens einem der vorhergehenden Ansprüche, **dadurch gekennzeichnet, dass** der Mischzylinder (1) ein Außengewinde (6) aufweist, welches mit einem Innengewinde (7) eines den Mischzylinder (1) teilweise umgebenden Hohlzylinders (5) in Eingriff steht, wobei der Hohlzylinder (5) mit dem Austragskolben (4) starr verbunden ist.

13. Vorrichtung nach Anspruch 12, **dadurch gekennzeichnet, dass** das der Austragsöffnung (3) gegenüberliegende Ende des Mischzylinders (1) im Wesentlichen offen ist und der aus dem offenen Ende herausragende Austragskolben (4) jenseits des Mischzylinders (1) mit dem den Mischzylinder (1) teilweise umgebenden Hohlzylinder (5) starr verbunden ist.

14. Vorrichtung nach Anspruch 12 oder 13, **dadurch gekennzeichnet, dass** der Hohlzylinder (5) an seiner Außenfläche im Bereich der starren Verbindung mit dem Austragskolben (4) Handhabungselemente aufweist.

15. Vorrichtung nach mindestens einem der Ansprüche 12 bis 14, **dadurch gekennzeichnet, dass** der Hohlzylinder (5) an seiner gesamten Außenfläche Handhabungselemente aufweist.

16. Vorrichtung nach mindestens einem der vorhergehenden Ansprüche, **dadurch gekennzeichnet, dass** die Vorrichtung im Bereich der Austragsöffnung (3) so mit einem Standfuß (20) verbindbar ist, dass die Austragsöffnung (3) dicht verschlossen ist.

17. Vorrichtung nach mindestens einem der vorhergehenden Ansprüche, **dadurch gekennzeichnet, dass** eine Kanüle (24) mit der Austragsöffnung (3) verbindbar ist.

18. Vorrichtung nach mindestens einem der vorhergehenden Ansprüche, **dadurch gekennzeichnet, dass** während des Mischvorgangs oder unmittelbar danach ein Unterdruck in dem Mischzylinder (1) erzeugbar ist.

19. Vorrichtung nach mindestens einem der vorhergehenden Ansprüche, **dadurch gekennzeichnet, dass** die Innenflächen der Lagerkammer (14) des Austragskolbens (4) und/oder des Mischzylinders (1) mit einer Schutzschicht überzogen sind.

## Claims

1. Device for mixing and delivery of liquid and powdery substances for medical use, with a mixing cylinder (1), with a perforated mixing plunger (8) which is axially and rotatably movable in the mixing cylinder (1) by means of an actuation rod (9), with a sealable delivery aperture (3) on the mixing cylinder (1), with an axially displaceable delivery plunger (4), with a means for actuating the delivery plunger (4) in order to deliver the mixed substances through the delivery aperture (3) by displacement of the delivery plunger (4) in the mixing cylinder (1), and with two chambers which can be separated from one another in a leaktight manner for the separate reception and storage of two substances to be mixed.
**characterized in that**
the delivery plunger (4) has, in its interior, a storage chamber (14) for receiving a substance to be mixed.

2. Device according to Claim 1, **characterized in that** two substances to be mixed can be received separately in the mixing cylinder (1) and in the storage chamber (14) in the interior of the delivery plunger (4).

3. Device according to Claim 2, **characterized in that** the delivery plunger (4) has a sealable aperture (17) via which, in the opened state, the substance located in the storage chamber (14) of the delivery plunger (4) can pass into the mixing cylinder (1).

4. Device according to Claim 3, **characterized in that** the sealable aperture (17) of the delivery plunger (4) can be actuated via a sealing mechanism arranged at that end of the delivery plunger (4) directed away from the mixing cylinder (1).

5. Device according to Claim 4, **characterized in that** the sealing mechanism has a sealing rod (18) which is mounted in an axially displaceable manner at that end of the delivery plunger (4) directed away from the mixing cylinder (1), and the sealable aperture (17) can be opened or closed by actuation of the sealing rod (18).

6. Device according to Claim 4 or 5, **characterized in that** the sealing mechanism has handling means for simple actuation.

7. Device according to at least one of the preceding claims, **characterized in that** the delivery plunger (4) has, at the end directed away from the mixing cylinder (1), a sealable filling aperture (15) for filling the storage chamber (14) of the delivery plunger (4) with one of the substances that are to be mixed.

8. Device according to at least one of the preceding claims, **characterized in that** the storage chamber (14) of the delivery plunger (4) is provided with vent apertures that permit a controlled filling and emptying of the storage chamber (14).

9. Device according to at least one of the preceding claims, **characterized in that** the delivery aperture (3) is arranged on an end face (2) of the mixing cylinder (1), and the actuation rod (9) of the mixing plunger (8) and the means for actuating the delivery plunger (4) are arranged at the opposite end of the mixing cylinder (1).

10. Device according to at least one of the preceding claims, **characterized in that** the mixing plunger (8) is arranged between the delivery aperture (3) and the delivery plunger (4) in the mixing cylinder (1), and the actuation rod (9) of the mixing plunger (8) is guided out through the delivery plunger (4) in a sealed manner.

11. Device according to at least one of the preceding claims, **characterized in that** the delivery plunger (4) engages with the mixing cylinder (1) via a thread, and, by rotation of the delivery plunger (4) relative to the mixing cylinder (1), the delivery plunger (4) can be displaced in the mixing cylinder, and the mixed substances located in the mixing cylinder can be dispensed.

12. Device according to at least one of the preceding claims, **characterized in that** the mixing cylinder (1) has an outer thread (6) which engages with an inner thread (7) of a hollow cylinder (5) that partially surrounds the mixing cylinder (1), the hollow cylinder (5) being rigidly connected to the delivery plunger (4).

13. Device according to Claim 12, **characterized in that that** end of the mixing cylinder (1) remote from the delivery aperture (3) is substantially open, and the delivery plunger (4) protruding from the open end is rigidly connected, on the other side of the mixing cylinder (1), to the hollow cylinder (5) that partially surrounds the mixing cylinder (1).

14. Device according to Claim 12 or 13, **characterized in that** the hollow cylinder (5) has handling elements on its outer surface, in the area of the rigid connection to the delivery plunger (4).

15. Device according to at least one of Claims 12 to 14, **characterized in that** the hollow cylinder (5) has handling elements over its entire outer surface.

16. Device according to at least one of the preceding claims, **characterized in that**, in the area of the delivery aperture (3), the device can be connected to a stand foot (20) in such a way that the delivery aperture (3) is tightly sealed.

17. Device according to at least one of the preceding claims, **characterized in that** a cannula (24) can be connected to the delivery aperture (3).

18. Device according to at least one of the preceding claims, **characterized in that** an underpressure can be generated in the mixing cylinder (1) during the mixing process or immediately thereafter.

19. Device according to at least one of the preceding claims, **characterized in that** the inner surfaces of the storage chamber (14) of the delivery plunger (4) and/or of the mixing cylinder (1) are covered with a protective layer.

## Revendications

1. Dispositif pour mélanger et distribuer des substances liquides et pulvérulentes pour l'utilisation médicale, avec un cylindre de mélange (1), avec un piston de mélange (8) percé, qui est mobile axialement et en rotation dans le cylindre de mélange (1) au moyen d'une tige d'actionnement (9), avec une ouverture de distribution refermable (3) sur le cylindre de mélange (1), avec un piston de distribution coulissant axialement (4), avec un moyen pour actionner le piston de distribution (4) pour distribuer les substances mélangées à travers l'ouverture de distribution (3) par un déplacement du piston de distribution (4) dans le cylindre de mélange (1), avec deux chambres séparables de façon étanche l'une de l'autre pour la réception et le stockage séparés de deux substances à mélanger, **caractérisé en ce que** le piston de distribution (4) présente intérieurement une chambre de stockage (14) pour recevoir une des substances à mélanger.

2. Dispositif selon la revendication 1, **caractérisé en ce que** deux substances à mélanger peuvent être contenues séparément dans le cylindre de mélange (1) et dans la chambre de stockage (14) à l'intérieur du piston de distribution (4).

3. Dispositif selon la revendication 2, **caractérisé en ce que** le piston de distribution (4) présente une ouverture refermable (17), par laquelle, lorsqu'elle est en position ouverte, la substance contenue dans la chambre de stockage (14) du piston de distribution (4) peut parvenir dans le cylindre de mélange (1).

4. Dispositif selon la revendication 3, **caractérisé en ce que** l'ouverture refermable (17) du piston de distribution (4) peut être actionnée par un mécanisme de fermeture disposé sur l'extrémité du piston de distribution (4) située à l'opposé du cylindre de mélange (1).

5. Dispositif selon la revendication 4, **caractérisé en ce que** le mécanisme de fermeture comprend une tige de fermeture (18) pouvant coulisser axialement montée sur l'extrémité du piston de distribution (4) située à l'opposé du cylindre de mélange (1), et l'ouverture refermable (17) peut être libérée ou fermée en actionnant la tige de fermeture (18).

6. Dispositif selon la revendication 4 ou 5, **caractérisé en ce que** le mécanisme de fermeture comprend des moyens de manipulation permettant un actionnement simple.

7. Dispositif selon au moins une des revendications précédentes, **caractérisé en ce que** le piston de distribution (4) présente, à l'extrémité située à l'opposé du cylindre de mélange (1), une ouverture de remplissage refermable (15) pour le remplissage de la chambre de stockage (14) du piston de distribution (4) avec une des substances à mélanger.

8. Dispositif selon au moins une des revendications précédentes, **caractérisé en ce qu'**il est prévu des ouvertures de ventilation de la chambre de stockage (14) du piston de distribution (4), qui permettent un remplissage contrôlé et une vidange contrôlée de la chambre de stockage (14).

9. Dispositif selon au moins une des revendications précédentes, **caractérisé en ce que** l'ouverture de distribution (3) est disposée dans une face d'extrémité (2) du cylindre de mélange (1) et la tige d'actionnement (9) du piston de mélange (8) et les moyens pour actionner le piston de distribution (4) sont disposés à l'extrémité opposée du cylindre de mélange (1).

10. Dispositif selon au moins une des revendications précédentes, **caractérisé en ce que** le piston de mélange (8) est disposé dans le cylindre de mélange (1) entre l'ouverture de distribution (3) et le piston de distribution (4) et la tige d'actionnement (9) du piston de mélange (8) est conduite de façon étanche vers l'extérieur à travers le piston de distribution (4).

11. Dispositif selon au moins une des revendications précédentes, **caractérisé en ce que** le piston de distribution (4) est assemblé au cylindre de mélange (1) via un filet, et le piston de distribution (4) peut coulisser dans le cylindre de mélange et les substances mélangées se trouvant dans le cylindre de mélange peuvent être distribuées par une rotation du piston de distribution (4) par rapport au cylindre de mélange (1).

12. Dispositif selon au moins une des revendications précédentes, **caractérisé en ce que** le cylindre de mélange (1) présente un filet extérieur (6), qui est en prise avec un filet intérieur (7) d'un cylindre creux (5) entourant partiellement le cylindre de mélange (1), dans lequel le cylindre creux (5) est solidaire du piston de distribution (4).

13. Dispositif selon la revendication 12, **caractérisé en ce que** l'extrémité du cylindre de mélange (1) opposée à l'ouverture de distribution (3) est essentiellement ouverte et le piston de distribution (4) sortant de l'extrémité ouverte de l'autre côté du cylindre de mélange (1) est solidaire du cylindre creux (5) entourant partiellement le cylindre de mélange (1).

14. Dispositif selon la revendication 12 ou 13, **caractérisé en ce que** le cylindre creux (5) comporte des éléments de manipulation sur sa surface extérieure, dans la région de la liaison solidaire avec le piston de distribution (4).

15. Dispositif selon au moins une des revendications 12 à 14, **caractérisé en ce que** le cylindre creux (5) comporte des éléments de manipulation sur toute sa surface extérieure.

16. Dispositif selon au moins une des revendications précédentes, **caractérisé en ce que** le dispositif peut être assemblé à un pied de montage (20) dans la région de l'ouverture de distribution (3), de telle manière que l'ouverture de distribution (3) soit fermée de façon étanche.

17. Dispositif selon au moins une des revendications précédentes, **caractérisé en ce qu'**une canule (24) peut être assemblée à l'ouverture de distribution (3).

18. Dispositif selon au moins une des revendications précédentes, **caractérisé en ce qu'**une dépression peut être produite dans le cylindre de mélange (1) pendant l'opération de mélange ou immédiatement après.

19. Dispositif selon au moins une des revendications précédentes, **caractérisé en ce que** les surfaces intérieures de la chambre de stockage (14) du piston de distribution (4) et/ou du cylindre de mélange (1) sont revêtues d'une couche de protection.
